(19) Europäisches Patentamt
European Patent Office
Office européen des brevets

(11) Numéro de publication : **0 352 168 B1**

# (12) FASCICULE DE BREVET EUROPEEN

(45) Date de publication du fascicule du brevet : **16.06.93 Bulletin 93/24**

(51) Int. Cl.$^5$ : **C07B 57/00,** C07C 59/68, C07C 51/43, C07C 51/42, C07C 51/487

(21) Numéro de dépôt : **89401971.0**

(22) Date de dépôt : **10.07.89**

## (54) Procédé de purification de l'acide D-hydroxyphénoxypropionique.

(30) Priorité : **20.07.88 FR 8809793**

(43) Date de publication de la demande : **24.01.90 Bulletin 90/04**

(45) Mention de la délivrance du brevet : **16.06.93 Bulletin 93/24**

(84) Etats contractants désignés : **AT BE CH DE ES FR GB GR IT LI LU NL SE**

(56) Documents cités :
**EP-A- 192 849**
**FR-A- 763 374**
**FR-A- 1 001 764**
**GB-A- 797 369**

(73) Titulaire : **RHONE-POULENC CHIMIE**
**25, quai Paul Doumer**
**F-92408 Courbevoie Cédex (FR)**

(72) Inventeur : **Moyne, José**
**4, Chemin de la Vire**
**F-69300 Caluire (FR)**

(74) Mandataire : **Ricalens, François et al**
**RHONE-POULENC CHIMIE Direction de la Propriété Industrielle 25, Quai Paul Doumer**
**F-92408 Courbevoie Cedex (FR)**

EP 0 352 168 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de l'acide D-hydroxyphénoxypropionique optiquement pur. Elle concerne plus particulièrement son procédé de purification.

Les esters aliphatiques de l'acide D-hydroxyphénoxypropionique optiquement pur sont des intermédiaires d'herbicides sélectifs très recherchés actuellement.

Ces esters peuvent être obtenus directement par inversion de Walden à partir de L chloro ou bromopropionate d'alkyle et d'hydroquinone en présence d'une base forte en milieu aqueux ou alcoolique. Malheureusement dans ces milieux les esters sont facilement hydrolysés et le motif chloropropionique subit une racémisation, tel que décrit par W.A Cowdrey, E.D Hargher et CH. K Ingold dans le "Journal of Chemical Society 1937, 1208.

Lorsque par exemple selon le brevet de la République Fédérale Allemande DE 3 150 233 on substitue le milieu eau ou alcool de l'article précédent par un milieu aprotique polaire tel que le diméthylsulfoxide ou le diméthylformamide on obtient une quantité importante de dérivés disubstitués de l'hydroquinone qu'il est difficile de séparer. Ce dérivé disubstitué consomme initialement deux modes de L-chloropropionate de méthyle, composé le plus onéreux qui n'est pas récupérable.

Il est aussi décrit dans le brevet européen n° 192 849 un procédé de préparation de l'acide 2-(4-hydroxyphénoxy)-propionique par réaction d'un acide chloro ou bromopropionique avec l'hydroquinone dans un milieu aqueux extrêmement concentré. En effet le rapport pondéral eau/hydroquinone est avantageusement compris entre 2 et 2,5. Le fait de travailler en milieu extrêmement concentré permet :

- de limiter la double substitution de l'hydroquinone
- d'augmenter la productivité car on peut transformer une quantité importante de l'hydroquinone introduite
- d'éviter la racémisation de l'acide D-chloropropionique ou de son sel de sodium.

Le fait de travailler en milieu concentré, à la limite de viscosité qui permet néanmoins une agitation suffisante, c'est-à-dire avec un rapport eau/hydroquinone d'environ 2,5, permet donc d'obtenir une meilleure sélectivité en acide D-hydroxyphénoxypropionique optiquement actif ou en son double sel de sodium. L'acide D-hydroxyphénoxypropionique optiquement actif et son sel de sodium contiennent une quantité importante d'hydroquinone qui est éliminée après acidification, tel que décrit dans le brevet EP 192 849, par extraction à la méthylisobutylcétone.

La phase aqueuse contenant l'acide hydroxyphénoxypropionique et son sel issue de cette opération d'extraction contient en poids environ 5 % de composés organiques indésirables pour 15 % d'acide D-hydroxyphénoxypropionique.

Le problème que ne résout pas la demande de brevet EP 192 849 est l'extraction de l'acide D-hydroxyphénoxypropionique pur à partir de ce mélange. L'acide D-hydroxyphénoxypropionique ne présente pas des critères de solubilité ou de cristallisation nettement différenciés de ceux du dérivé disubstitué ni de ceux des dérivés alkylés directement sur le carbone du noyau aromatique de l'hydroquinone. L'extraction du produit pur posait donc un problème difficile à résoudre pour l'industrie.

La présente invention a permis d'atteindre cet objectif, la préparation de l'acide D-hydroxyphénoxypropionique et de ses esters purs.

La présente invention consiste en un procédé de purification d'acide D- hydroxyphénoxypropionique optiquement pur à partir d'un milieu réactionnel issu de la réaction d'acides chloro ou bromo propionique sur de l'hydroquinone en présence d'une base choisie parmi la soude et la potasse, milieu contenant des sels minéraux, des dérivés alkylés ou alcoxylés de l'hydroquinone, de l'acide hydroxyphénoxypropionique racémique caractérisé en ce qu'on acidifie le milieu à pH environ 1 pour précipiter l'acide hydroxyphénoxypropionique et les dérivés de l'hydroquinone, on élimine une partie de la phase aqueuse contenant les sels minéraux, on ajoute de l'eau et on dissout par chauffage l'acide hydroxyphénoxypropionique et les dérivés de l'hydroquinone, on laisse refroidir lentement de façon à laisser précipiter les cristaux d'acide D- hydroxyphénoxypropionique optiquement pur enrobés par une huile contenant les différentes impuretés, on lave par élution les dits cristaux avec de l'eau.

Le procédé consiste à partir du L chloropropionate de méthyle à préparer le L chloropropionate de sodium par hydrolyse avec une base alcaline selon la réaction.

$$CH_3 - \overset{\bullet}{C}HCl-COO\ CH_3 + H_2O \xrightarrow{MOH} CH_3\ \overset{\bullet}{C}HCl - COOH + CH_3OH$$

dans laquelle M représente un métal alcalin choisi parmi le sodium ou le potassium.

L'alcool et une partie de l'eau sont éliminés par distillation.

L'acide L- chloropropionique sous forme de son sel alcalin obtenu précédemment est mis en contact avec le double sel de sodium de l'hydroquinone pour former le double sel de l'acide D-hydroxyphénoxypropionique.

L'équation peut être schématisée de la façon suivante :

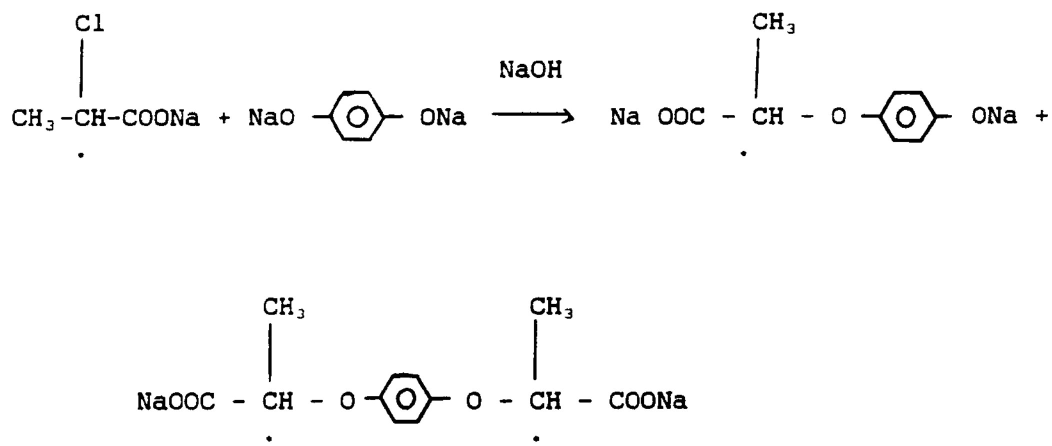


Le double sel de sodium de l'hydroquinone est formé par introduction d'hydroquinone de préférence selon un rapport pondéral compris entre 1 et 1,5 fois le poids de soude.

On additionne alors la solution aqueuse d'acide L-chloropropionique sous forme de son sel de sodium à la suspension sodique d'hydroquinone de façon à ce que le rapport pondéral entre l'eau totale du mélange et l'hydroquinone soit compris entre 2,4% et 2,8%. En dessous de ce rapport le milieu n'est pas agitable car il est trop visqueux, au dessus de ce rapport la condensation provoque une grande quantité de dérivés disubstitués.

La température de réaction est maintenue notamment entre 30 et 50°C.

La quantité molaire d'hydroquinone introduite par rapport au L-chloropropionate est comprise de préférence entre 1,2 et 1,5. On travaille donc en excès molaire d'hydroquinone.

En fin de réaction on acidifie le milieu à pH environ 6 de façon à libérer l'hydroquinone de son sel. Le milieu devient homogène.

L'hydroquinone est extraite du milieu réactionnel par un solvant acétonique et de préférence par la méthylisobutylcétone.

La solution aqueuse résiduaire est distillée de façon à éliminer la cétone en solution dans la phase aqueuse puis acidifiée à pH inférieur à 1 de façon à précipiter l'acide hydroxyphénoxypropionique brut, avantageusement, la quantité d'eau qui permet l'élution de l'huile est comprise entre 0,6 et 0,8 fois le poids des cristaux d'acide D-hydroxyphénoxypropionique.

Ainsi selon un des modes préférés de l'invention, il est possible de purifier de l'acide D-hydroxyphénoxy propionique obtenu par action de l'hydroquinone sur du L-chloro ou bromo propionate d'alkyle en 9 étapes:

- . dans une 1è étape on hydrolyse le L-chloro ou bromopropionate d'alkyle dans l'eau en présence d'une base forte et on élimine l'alcool formé par distillation.
- . dans une 2è étape on prépare une suspension alcaline aqueuse d'hydroquinone,
- . dans une 3è étape, on met en contact la suspension alcaline aqueuse d'hydroquinone et la solution d'acide L-chloro ou bromopropionique sous forme de son sel de sodium de façon à ce que la quantité totale d'eau du milieu soit incluse dans un rapport pondéral calculé par rapport à l'hydroquinone compris entre 2,4 % et 2,8 %,
- . dans une 4è étape on acidifie entre pH 4 et 6 le milieu et on extrait l'hydroquinone par un solvant cétonique qui peut être recyclé à la 2è étape,
- . dans une 5è étape on acidifie la solution aqueuse résiduaire à pH environ 1 de façon à précipiter l'acide hydroxyphénoxypropionique,
- . dans une 6è étape on élimine une partie de la phase aqueuse par décantation et on lave le précipité avec une faible quantité d'eau,
- . dans une 7è étape on chauffe le précipité dans l'eau de façon à le solubiliser et on refroidit lentement le milieu jusqu'à environ la température ambiante,
- . dans une 8è étape on sépare par filtration l'acide D-hydroxyphé-noxypropionique enrobé par une huile

contenant les différentes impuretés,

. dans une 9è étape on lave l'acide D-hydroxyphénoxypropionique par élution à l'eau.

La suspension aqueuse contenant ledit précipité a approximativement la composition suivante calculée par rapport à l'acide hydroxyphénoxypropionique recherché (HPPA)

| | | |
|---|---|---|
| Hydroquinone | = | 0,7 à 1 % |
| Bisalcoxyhydroquinone | = | 10 à 15 % |
| Composés de l'hydroquinone alkylés sur le noyau | = | 2 à 4 % |
| Acides aliphatiques | = | 7 à 10 % |
| NaCl | = | 200 % |
| HCl | = | 7 à 10 % |

La présente invention a pour objet un procédé de purification de l'acide D-hydroxyphénoxypropionique à partir du milieu de préparation de ce composé.

Il consiste dans une première étape à éliminer par décantation une partie de la phase aqueuse contenant les sels minéraux et à laver deux à trois fois le précipité d'acide hydroxyphénoxypropionique avec le minimum d'eau possible. La quantité d'eau de lavage est de préférence égale à 2 à 3 fois le poids d'acide hydroxyphénoxypropionique ; ces lavages permettent de diluer la teneur en sels minéraux d'environ la moitié.

Dans une deuxième étape on ajoute une quantité d'eau de façon à ce que la quantité totale d'eau soit comprise entre 2 et 4 fois le poids d'acide hydroxyphénoxypropionique, on chauffe le milieu à une température comprise entre 90 et 100°C de façon à solubiliser tous les composants et on refroidit lentement jusqu'à la température ambiante. Le milieu se sépare en trois phases :

- une phase solide contenant l'acide D-hydroxyphénoxypropionique
- une phase huileuse contenant l'hydroquinone, l'acide hydroxyphénoxypropionique racémique, le dérivé bisalkoxylé de l'hydroquinone
- une phase aqueuse

Dans une troisième étape, on filtre les cristaux d'acide D-hydroxyphénoxypropionique qui sont entourés d'une couche huileuse contenant les différentes impuretés. Ensuite, on déplace cette huile par élution avec de l'eau. Ce lavage est réalisé par élution pour "pousser" l'huile à travers le filtre en provoquant le minimum de turbulence au sein du gâteau de filtration. L'huile est ainsi déplacée dans un filtre du type "piston" avec une quantité d'eau minimale de façon à ne pas dissoudre l'acide, cette quantité est avantageusement comprise entre 0,6 et 0,8 fois le poids du gâteau humide.

Par cette technique de purification il est possible d'obtenir l'acide D-hydroxyphénoxypropionique avec un rendement de purification et de récupération à partir du milieu de synthèse supérieur à 90 %, l'acide hydroxyphénoxypropionique présentant une pureté optique supérieure à 98 % en dérivé dextrogyre. L'acide D-hydroxyphénoxypropionique est ensuite estérifié selon toute méthode connue de l'homme de l'art.

La présente invention sera plus complètement décrite à l'aide de l'exemple ci-joint qui ne doit pas être considéré comme limitatif de l'invention.

## Exemple

Dans un réacteur de 50 litres muni d'une colonne à distiller on introduit en pied 12,250 kg de L-chloropropionate de méthyle (100 moles) et l'on additionne, à une température de 30-35°C, 19,080 kg de soude à 21,4 % en 1 heure ; on maintient 1/2 heure à cette température pour finir d'hydrolyser l'ester, puis on distille 6,350 kg d'un mélange à 50 % d'eau et de méthanol.

Cette solution concentrée de L chloropropionate de sodium est utilisée dans les 24 heures.

Dans un réacteur de 100 litres en acier émaillé on introduit 10,6 kg de soude (265 moles) sous forme de soude aqueuse de 36 % à 26 %.

On additionne en 1 heure 13,750 kg (125 moles) d'hydroquinone et l'on maintient la température à 40°C par refroidissement.

Si l'on est trop concentré, l'hydroquinonate formé n'est pas agitable, et si l'on est trop dilué on augmente la teneur en composé bisubstitué.

Sur cette suspension d'hydroquinonate de sodium dans l'eau, on ajoute en 4 -5 heures à 40°C la solution aqueuse de L chloropropionate de sodium préparée ci-dessus. On maintient de 1 à 3 heures à 40°C jusqu'à ce que la teneur en acide hydroxyphénoxypropionique ne grandisse plus (suivi de la réaction en chromatographie phase liquide).

Par addition d'acide chlorhydrique concentré, on amène le pH à une valeur de 6 et l'on extrait l'hydroquinone en excès par 3 extractions avec de la méthylisobutylcétene soit au total 36,9 kg de méthylisobutylcétone.

La phase aqueuse est distillée pour enlever la méthylisobutylcétone soluble puis acidifiée à pH < 1 pour précipiter le D-HPPA brut.

Poids du milieu réactionnel 86 kg contenant :

| | | |
|---|---|---|
| Hydroquinone | : | 0,100 kg |
| Acide hydroxyphénoxypropionique | : | 13,286 kg - 73 moles |
| | | (dont 3 ou 4 de racémique) |
| Dérivés bisalkoxyles | : | 1,524 kg - 6 moles |
| Dérivés C alkylés | : | 0,254 kg |
| NaCl | : | 21,469 kg |
| HCl | : | 0,934 kg |
| Acides aliphat. | : | 1,140 kg |
| $H_2O$ | : | 47,301 kg |
| TOTAL | | 86,008 kg |

On laisse décanter le précipité d'acide hydroxyphénoxypropionique brut, on soutire 26,877 kg de phase aqueuse saturée de NaCl; on remet 8,2 kg d'eau, on agite et on laisse décanter. On soutire de nouveau 13,750 kg de phase aqueuse et l'on rajoute 21,844 kg d'eau dans le réacteur.

Le milieu a alors la composition suivante :

| | | |
|---|---|---|
| Hydroquinone | : | 0,070 kg |
| Acide hydroxyphénoxypropionique | : | 12,847 kg - 70,6 moles |
| | | - Isomère D + Racémique |
| Dérivés bisalcoxylés | : | 1,270 kg - 5 moles |
| Dérivés C alkylés | : | 0,250 kg |
| NaCl | : | 10,080 kg |
| HCL | : | 0,439 kg |
| $H_2O$ | : | 49,919 kg |
| Acides aliphatiques | : | 0,550 kg |
| TOTAL | | 75,425 kg |

Le milieu est chauffé à 95°C pour solubiliser entièrement les divers composants puis on descend très lentement la température de 90 à 70°C. Le milieu se trouble, devient laiteux montrant l'apparition d'une huile et

non de cristaux. A partir de 70°C et jusqu'à 20°C il y a apparition de jolis cristaux d'acide hydroxyphénoxypropionique dextrogyre.

Par filtration sous pression, on élimine dans les jus mères une partie de l'huile mais l'essentiel est éliminé du précipité physiquement en la déplaçant par le moins d'eau possible soit environ 9 kg d'eau.

Si l'on agite pas les jus de lavages pour ne pas dissoudre l'huile totalement ou partiellement dans l'eau, on peut l'analyser par chromatographie liquide haute pression et constater que l'on a un rapport presque constant entre les divers constituants :

| | | en moles |
|---|---|---|
| Hydroquinone | : | 1 % |
| Acide hydroxyphénoxypropionique | : | 54 % (en partie racémique) |
| Dérivés C alkylés | : | 8 % (Il y a 3 composés différents) |
| Dérivés bisalkoxylés | : | 37 % (essentiellement le stéréoisomère D-D) |

Poids du gâteau séché sous vide à 80°C : 11,766 kg (64,6 moles)

Pureté en acide hydroxyphenoxypropionique : 99 %

Teneur en dérivés bisalkoxylés $\leq$ 1 %

Excès enantiomérique : 98 à 99 %

(chromatographie sur colonne chirale)

Remarques :

Sur 70 moles d'acide hydroxyphénoxypropionique présentes en fin de synthèse on en retrouve 64,6 dans le produit final.

Soit un rendement de récupération de : 92 %

Alors que le traitement nous a permis d'éliminer 5,5 moles de dérivés bisalkoxylés, 3 à 4 moles d'acide hydroxyphénoxypropionique racémique et l'équivalent d'une mole des 3 produits de C alkylation.

A partir de cet acide D-hydroxyphénoxypropionique pur il est possible d'obtenir directement des esters purs chimiquement et optiquement sans problème de séparation.

## Revendications

1. Procédé de purification d'acide D- hydroxyphénoxypropionique optiquement pur à partir d'un milieu réactionnel issu de la réaction d'acides chloro ou bromo propionique sur de l'hydroquinone en présence d'une base choisie parmi la soude et la potasse, milieu contenant des sels minéraux, des dérivés alkylés ou alcoxylés de l'hydroquinone, de l'acide hydroxyphénoxypropionique racémique caractérisé en ce qu'on acidifie le milieu à pH environ 1 pour précipiter l'acide hydroxyphénoxypropionique et les dérivés de l'hydroquinone, on élimine une partie de la phase aqueuse contenant les sels minéraux, on ajoute de l'eau et on dissout par chauffage l'acide hydroxyphénoxypropionique et les dérivés de l'hydroquinone, on laisse refroidir lentement de façon à laisser précipiter les cristaux d'acide D-hydroxyphénoxypropionique optiquement pur enrobés par une huile contenant les différentes impuretés, on lave par élution les dits cristaux avec de l'eau.

2. Procédé selon la revendication 1 caractérisé en ce que le chauffage permettant la dissolution de l'acide hydroxyphénoxypropionique et des dérivés de l'hydroquinone est réalisé entre 90 et 100°C.

3. Procédé selon la revendication 1 caractérisé en ce que la quantité d'eau permettant la dissolution de l'acide D-hydroxyphénoxypropionique est comprise entre 2 et 4 fois le poids de ce dernier.

4. Procédé selon la revendication 1 caractérisé en ce que la quantité d'eau qui permet l'élution de l'huile est comprise entre 0,6 et 0,8 fois le poids des cristaux d'acide D-hydroxyphénoxypropionique.

5. Procédé de préparation de purification de l'acide D-hydroxyphénoxypropionique optiquement pur à partir d'hydroquinone et de L-chloro ou bromopropionate d'alkyle en 9 étapes selon lesquelles :
   . dans une 1è étape on hydrolyse le L-chloro ou bromopropionate d'alkyle dans l'eau en présence d'une base forte et on élimine l'alcool formé par distillation,
   . dans une 2è étape on prépare une suspension alcaline aqueuse d'hydroquinone,
   . dans une 3è étape, on met en contact la suspension alcaline aqueuse d'hydroquinone et la solution d'acide L-chloro ou bromopropionique sous forme de son sel de sodium de façon à ce que la quantité totale d'eau du milieu soit incluse dans un rapport pondéral calculé par rapport à l'hydroquinone compris entre 2,4 % et 2,8 %,
   . dans une 4è étape on acidifie entre pH 4 et 6 le milieu et on extrait l'hydroquinone par un solvant cétonique qui peut être recyclé à la 2è étape,
   . dans une 5è étape on acidifie la solution aqueuse résiduaire à pH environ 1 de façon à précipiter l'acide hydroxyphénoxypropionique,
   . dans une 6è étape on élimine une partie de la phase aqueuse par décantation et on lave le précipité avec une faible quantité d'eau,
   . dans une 7è étape on chauffe le précipité dans l'eau de façon à le solubiliser et on refroidit lentement le milieu jusqu'à environ la température ambiante,
   . dans une 8è étape on sépare par filtration l'acide D-hydroxyphénoxypropionique enrobé par une huile contenant les différentes impuretés,
   . dans une 9è étape on lave l'acide D-hydroxyphénoxypropionique par élution à l'eau.

## Claims

1. Process for the purification of optically pure D-hydroxyphenoxypropionic acid from a reaction medium resulting from the reaction of chloropropionic or bromopropionic acid on hydroquinone in the presence of a base chosen from among soda and potash, the medium containing mineral salts, alkyl or alkoxyl hydroquinone derivatives and racemic hydroxyphenoxypropionic acid, characterized in that the medium is acidified to a pH of approximately 1 for precipitating the hydroxyphenoxypropionic acid and the hydroquinone derivatives, part of the aqueous phase containing the mineral salts is eliminated, water is added and the hydroxyphenoxypropionic acid and the hydroquinone derivatives are dissolved by heating, the optically pure D-hydroxyphenoxypropionic acid crystals coated with an oil containing the different impurities are slowly cooled so as to allow precipitation to take place, followed by the washing by elution of said crystals with water.

2. Process according to claim 1, characterized in that the heating permitting the dissolving of the hydroxyphenoxypropionic acid and the hydroquinone derivatives is carried out at between 90 and 100°C.

3. Process according to claim 1, characterized in that the water quantity permitting the dissolving of the D-hdydroxyphenoxypropionic acid is between 2 and 4 times the weight of the latter.

4. Process according to claim 1, characterized in that the water quantity permitting the elution of the oil is between 0.6 and 0.8 times the weight of the D-hydroxyphenoxypropionic acid crystals.

5. Process for the purification of optically pure D-hydroxyphenoxypropionic acid from hydroquinone and alkyl-L-chloropropionate or bromopropionate in nine stages according to which:
   in a first stage the alkyl-L-chloropropionate or bromopropionate is hydrolyzed in water in the presence of a strong
   base and the alcohol formed is eliminated by distillation.
   in a second stage an aqueous alkaline hydroquinone suspension is prepared,

in a third stage contacting takes place between the aqueous alkaline hydroquinone suspension and the L-chloropropionic or L-bromopropionic acid solution in the form of its sodium salt, so that the total water quantity in the medium is in a weight ratio calculated with respect to the hydroquinone between 2.4 and 2.8%,
in a fourth stage acidification takes place to between pH 4 and 6 of the medium and the hydroquinone is extracted by a ketone solvent, which can be recycled in the second stage,
in a fifth stage acidification takes place of the residual aqueous solution to a pH of approximately 1, so as to precipitate the hydroxyphenoxypropionic acid,
in a sixth stage elimination takes place of part of the aqueous phase by settling and the precipitate is washed with a small amount of water,
in a seventh stage the precipitate is heated in water so as to solubilize it and slow cooling takes place of the medium to roughly ambient temperature,
in an eighth stage the oil-coated D-hydroxyphenoxypropionic acid containing the different impurities is separated by filtration and
in a ninth stage the D-hydroxyphenoxypropionic acid is washed by elution with water.

**Patentansprüche**

1. Verfahren zur Reinigung von optisch reiner D-Hydroxyphenoxypropionsäure ausgehend von einem Reaktionsgemisch, das der Reaktion von Chlor- oder Brompropionsäuren mit Hydrochinon in Gegenwart einer unter Natriumhydroxid- und Kaliumhydroxid-Lösung ausgewählten Base entstammt, und das Mineralsalze, alkylierte oder alkoxylierte Hydrochinonderivate und racemische Hydroxyphenoxypropionsäure enthält, dadurch gekennzeichnet, daß das Gemisch auf einen pH-Wert von ungefähr 1 angesäuert wird, um die Hydroxyphenoxypropionsäure und die Hydrochinonderivate auszufällen, daß ein Teil der die Mineralsalze enthaltenden wässrigen Phase entfernt und Wasser zugegeben wird, daß durch Erhitzen die Hydroxyphenoxypropionsäure und die Hydrochinonderivate wieder in Lösung gebracht werden, daß langsam abgekühlt wird, um Kristalle von optisch reiner D-Hydroxyphenoxypropionsäure, die von einem die verschiedenen Verunreinigungen enthaltenden Öl umhüllt sind, auszufällen und daß die besagten Kristalle durch Elution mit Wasser gewaschen werden.

2. Verfahren nach Anspruch 1, dadurch gekennzeichnet, daß das Erwärmen, das das Auflösen der Hydroxyphenoxypropionsäure und der Hydrochinonderivate ermöglicht, zwischen 90 und 100°C durchgeführt wird.

3. Verfahren nach Anspruch 1, dadurch gekennzeichnet, daß die Menge Wasser, die das Auflösen der D-Hydroxyphenoxypropionsäure ermöglicht, das 2- bis 4-fache des Gewichtes der letzteren beträgt.

4. Verfahren nach Anspruch 1, dadurch gekennzeichnet, daß die Menge Wasser, die die Elution des Öls ermöglicht, das 0,6- bis 0,8-fache des Gewichts der Kristalle der D-Hydroxyphenoxypropionsäure beträgt.

5. Verfahren zur Reinigung von optisch reiner D-Hydroxyphenoxypropionsäure ausgehend von Hydrochinon und L-Chlor- oder Brompropionsäurealkylester in 9 Stufen, demzufolge :
   - in einem ersten Schritt L-Chlor- oder Brompropionsäurealkylester in Wasser in Gegenwart einer starken Base hydrolysiert und der dabei gebildete Alkohol durch Destillation abgetrennt wird,
   - in einem zweiten Schritt eine alkalische wässrige Suspension von Hydrochinon hergestellt wird,
   - in einem dritten Schritt die alkalische wässrige Suspension des Hydrochinons zu der Lösung der L-Chlor- oder Brompropionsäure in Gestalt ihres Natriumsalzes zugegeben wird, so daß die Gesamtmenge Wasser des Reaktionsgemisches bezogen auf das Hydrochinon in einem Gewichtsverhältnis von 2,4 bis 2,8 % vorliegt,
   - in einem 4. Schritt das Reaktionsgemisch auf pH 4 bis 6 angesäuert wird, um das Hydrochinon mit Hilfe eines ketonischen Lösemittels, das im zweiten Schritt zurückgewonnen werden kann, zu extrahieren,
   - in einem 5. Schritt die zurückbleibende wässrige Lösung ungefähr auf pH 1 angesäuert wird, um die Hydroxyphenoxypropionsäure auszufällen,
   - in einem 6. Schritt ein Teil der wässrigen Phase durch Dekantieren abgetrennt wird, und der Niederschlag mit einer geringen Menge Wasser gewaschen wird,
   - in einem 7. Schritt der Niederschlag in Wasser erhitzt wird, so daß er in Lösung geht, und das Gemisch langsam bis auf Raumtemperatur abgekühlt wird,

- in einem 8. Schritt die von einem die verschiedenen Verunreinigungen enthaltenden Öl umhüllte D-Hydroxyphenoxypropionsäure durch Filtration abgetrennt wird,
- in einem 9. Schritt die D-Hydroxyphenoxypropionsäure durch Elution mit Wasser gewaschen wird.